Europäisches Patentamt
European Patent Office
Office européen des brevets

(19)

(11) Veröffentlichungsnummer: **0 262 085 A1**

(12)

# EUROPÄISCHE PATENTANMELDUNG

(21) Anmeldenummer: **87810454.6**

(22) Anmeldetag: **10.08.87**

(51) Int. Cl.⁴: **C 07 D 471/18**
C 12 P 17/18, A 61 K 31/495
//(C07D471/18,241:00,221:00,
221:00),(C12P17/18,C12R1:01)

(30) Priorität: **15.08.86 CH 3294/86**

(43) Veröffentlichungstag der Anmeldung:
**30.03.88 Patentblatt 88/13**

(84) Benannte Vertragsstaaten:
**AT BE CH DE ES FR GB GR IT LI LU NL SE**

(71) Anmelder: **Gesellschaft für Biotechnologische Forschung mbH (GBF)**
**Mascheroder Weg 1**
**D-3300 Braunschweig-Stöckheim (DE)**

**CIBA-GEIGY AG**
**Klybeckstrasse 141**
**CH-4002 Basel (CH)**

(72) Erfinder: **Reichenbach, Hans, Prof. Dr.**
**Platanenstrasse 35**
**D-3340 Wolfenbüttel (DE)**

**Gerth, Klaus, Dr.**
**Am Butterbusch 17**
**D-3300 Braunschweig (DE)**

**Irschik, Herbert, Dr.**
**Masurenweg 15**
**D-3340 Wolfenbüttel (DE)**

**Kunze, Brigitte, Dr.**
**Hinter Aegidien 5**
**D-3300 Braunschweig (DE)**

**Höfle, Gerhard, Prof. Dr.**
**Am Windmühlenwerg 2**
**D-3300 Braunschweig (DE)**

**Augustiniak, Hermann, Dr.**
**In den Lindendoehren 9**
**D-3340 Wolfenbüttel (DE)**

**Bedorf, Norbert, Dr.**
**Krukenbergstrasse 4**
**D-3308 Königslutter (DE)**

**Jansen, Rolf, Dr.**
**Falkenbergstrasse 14**
**D-3300 Braunschweig (DE)**

**Trowitzsch-Kienast, Wolfram, Dr.**
**Im Dorfe 19**
**D-3300 Braunschweig (DE)**

**Steinmetz, Heinrich**
**Schildweg 44**
**D-3320 Hildesheim-Sorsum (DE)**

Der (Die) Mikroorganismus (Mikroorganismen) ist (sind) bei der National Collection of Industrial & Marine Bacteria unter der Nummer NCIB 12268 hinterlegt worden.

(54) **Antibiotika aus Myxococcus.**

(57) Die Erfindung betrifft neue polycyclische Alkaloide der Formel

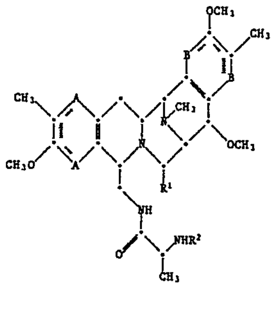

(I),

worin $R^1$ Wasserstoff oder Hydroxy, $R^2$ Wasserstoff oder Acyl und A und B unabhängig voneinander C=O oder C-OH bedeuten, wobei die gestrichelte Linie eine C=C-Doppelbindung an der Stelle der mittleren Bindung, wenn A oder B C=O sind, oder an der Stelle der beiden äusseren Bindungen, wenn A oder B C-OH sind, symbolisiert, ein Verfahren zur Herstellung dieser Verbindungen durch Fermentation eines neuen Mikroorganismus der Spezies Myxococcus xanthus, den neuen Mikroorganismus selbst, pharmazeutische Präparate, die die neuen Verbindungen enthalten, und die Verwendung der neuen Verbindungen als Antibiotika und als tumorhemmende Mittel und zur Herstellung von pharmazeutischen Präparaten.

EP 0 262 085 A1

**Beschreibung**

Antibiotika aus Myxococcus

Die Erfindung betrifft neue polycyclische Alkaloide mit anitbiotischen Eigenschaften, ein Verfahren zur Herstellung dieser Verbindungen durch Fermentation eines neuen Mikroorganismus der Spezies Myxococcus xanthus, den neuen Mikroorganismus selbst, pharmazeutische Präparate, die die neuen Verbindungen enthalten, und die Verwendung der neuen Verbindungen als Antibiotika und als tumorhemmende Mittel und zur Herstellung von pharmazeutischen Präparaten.

Die Erfindung betrifft insbeondere Verbindungen der Formel

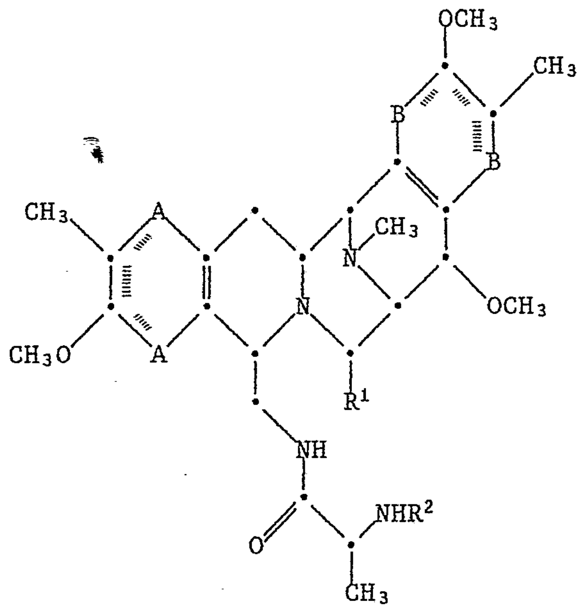

(I),

worin $R^1$ Wasserstoff oder Hydroxy, $R^2$ Wasserstoff oder Acyl und A und B unabhängig voneinander C=O oder C-OH bedeuten, wobei die gestrichelte Linie eine C=C-Doppelbindung an der Stelle der mittleren Bindung, wenn A oder B C=O sind, oder an der Stelle der beiden äusseren Bindungen, wenn A oder B C-OH sind, symbolisiert, und Salze solcher Verbindungen, insbesondere pharmazeutisch verwendbare Salze.

Die erfindungsgemässen Verbindungen der Formel I weisen nahe Verwandtschaft mit den aus Streptomyces lavendulae isolierten Verbindungen auf, die Saframycine genannt werden. In U.S. Patent 4 248 863 ist beispielsweise die fermentative Herstellung der Saframycine A, B, C, D und E, in U.S. Patent 4 372 947 die Herstellung von Saframycin S beschrieben. Die Struktur der bekannten Saframycine ist in T. Arai, Antimicrobial Agents and Chemotherapy 28, 5 (1985) angeführt. Demnach unterscheiden sich die erfindungsgemässen Verbindungen der Formel I von den vorbekannten Saframycinen zumindest durch den Alanyl-Rest in der Seitenkette, an dessen Stelle in den vorbekannten Saframycinen der Acylrest der Brenztraubensäure oder ein anderer Acylrest steht, ferner zusätzlich auch noch durch die Bedeutung des Restes $R^1$ und durch die Methoxygruppe am überbrückten bicyclischen Kern. Saframycin-Derivate mit einem Alanyl-Rest in der Seitenkette sind in der Europäischen Patentanmeldung EP 173 649 beschrieben, unterscheiden sich aber von den erfindungsgemässen Verbindungen in der Bedeutung des Restes $R^1$ und durch eine fehlende Methoxygruppe.

In der Europäischen Patentanmeldung EP 55 299 sind die Antibiotica Y-16482 $\alpha$ und $\beta$ beschrieben, die durch Fermentation eines Stammes von Pseudomonas fluorescens erhalten werden. Diese Verbindungen, auch Safracine genannt, weisen nach Y. Ikeda et al., J. Antibiotics 36, 1284 (1983) nahe Verwandtschaft mit den Saframycinen und damit auch mit den erfindungsgemässen Verbindungen der Formel I auf. Die Safracine unterscheiden sich jedoch von den vorliegenden neuen Verbindungen durch die Bedeutung von B (eine der Gruppen B ist C-H) und das Fehlen der Methoxygruppe am überbrückten bicyclischen Kern.

Weitere nah verwandte Verbindungen können aus dem Schwamm Reniera sp. isoliert werden. Diese von J.M. Frinke und D.J. Faulkner, J. Am. Chem. Soc. 104, 265 (1982) Renieramycine genannten Verbindungen unterscheiden sich von den erfindungsgemässen Verbindungen der Formel I zumindest durch die Seitenkette, die statt Alanylaminomethyl in den Renieramycinen (Z)-2-Methyl-2-butenoyloxymethyl (ein Angelicasäureester) ist.

Die Konfiguration an den chiralen Zentren der erfindungsgemässen Verbindungen der Formel I ist nicht mit Sicherheit bekannt. Hinweise für die relative Konfiguration an den verschiedenen chiralen Zentren ergeben sich allerdings aus dem "Nuclear Overhauser Effect" in Protonen-Kernresonanzspektren und aus der Analyse des bei der Hydrolyse freigesetzten Alanins. In Analogie zur bekannten, durch Röntgenstrukturanalyse

bestimmten absoluten Konfiguration von Saframycin C (T. Arai et al., Tetrahedron Letters 1979, 2355) und von bromiertem Safracin A (I. Ueda et al., Acta Cryst. C 40 1578 (1984)) ist anzunehmen, dass die erfindungsgemässen Verbindungen die räumliche Struktur gemäss Formel

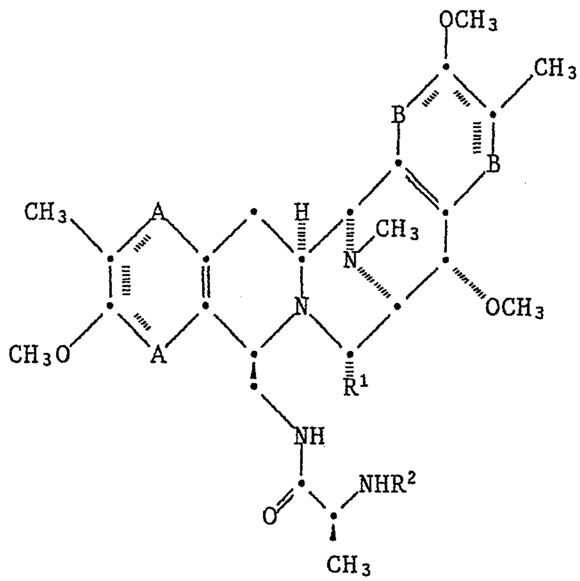

(II)

aufweisen.

In Verbindungen der Formel I und II bedeutet $R^2$ Wasserstoff oder Acyl. Acyl $R^2$ ist in erster Linie die Acylgruppe einer Carbonsäure oder eines Kohlensäurehalbesters mit bis zu 20 Kohlenstoffatomen, beispielsweise $C_1$-$C_{20}$-Alkanoyl, durch Hydroxy, Oxo oder Halogen substituiertes $C_2$-$C_7$-Alkanoyl, Aroyl oder $C_1$-$C_7$-Alkoxycarbonyl. $C_1$-$C_{20}$-Alkanoyl ist vorzugsweise $C_1$-$C_7$-Alkanoyl, beispielsweise Formyl, Acetyl, Propionyl, n-Butyryl, 2-Methylpropionyl, n-Pentanoyl, 2,2-Dimethylpropionyl, 2-Methylbutyryl, 3-Methylbutyryl oder n-Hexanoyl, oder geradkettiges $C_8$-$C_{20}$-Alkanoyl mit einer geraden Anzahl Kohlenstoffatome, beispielsweise n-Octanoyl, n-Decanoyl, n-Dodecanoyl, n-Tetradecanoyl, n-Hexadecanoyl, n-Octadecanoyl oder n-Icosanoyl. Durch Hydroxy, Oxo oder Halo substituiertes $C_2$-$C_7$-Alkanoyl ist beispielsweise Trifluoracetyl, Mono-, Di- oder Trichloracetyl, Glykoloyl, Glyceroyl, Lactoyl, Glyoxyloyl oder Pyruvoyl. Aroyl ist beispielsweise Benzoyl oder 1- oder 2-Naphthoyl, worin der Phenyl- oder Naphthylring durch Nitro, Amino, Halogen, beispielsweise Chlor oder Brom, Hydroxy und/oder $C_1$-$C_4$-Alkoxy, beispielsweise Methoxy, substituiert sein kann, beispielsweise 4-Nitrobenzoyl, 3,5-Dinitrobenzoyl, Anthraniloyl, 2,6-Dichlorbenzoyl, Salicyloyl, Galloyl oder 2-, 3- oder 4-Anisoyl. $C_1$-$C_7$-Alkoxy-carbonyl ist beispielsweise Methoxy-, Ethoxy-, n-Propoxy-, Isopropoxy-, n-Butoxy-, Isobutoxy- oder tert-Butoxy-carbonyl.

Bevorzugter Rest Acyl $R^2$ ist $C_1$-$C_7$-Alkanoyl, insbesondere Acetyl.

Bevorzugt sind die Verbindungen der Formel I, worin $R^1$ Wasserstoff oder Hydroxy, $R^2$ Wasserstoff, A C=O und B C=O oder C-OH bedeuten und die gestrichelte Linie die obengenannte Bedeutung hat, und pharmazeutisch verwendbare Salze solcher Verbindungen. Ebenfalls bevorzugt sind die Verbindungen der Formel I, worin $R^1$ Hydroxy, $R^2$ Wasserstoff, A C-OH und B C-OH bedeuten und die gestrichelte Linie die obengenannte Bedeutung hat, ferner die Verbindung der Formel I, worin $R^1$ Hydroxy, $R^2$ Acetyl, A C=O und B C=O bedeuten und die gestrichelte Linie die obengenannte Bedeutung hat, und pharmazeutisch verwendbare Salze dieser Verbindungen.

In erster Linie betrifft die Erfindung Verbindung der Formel I, worin $R^1$ Wasserstoff oder Hydroxy, $R^2$ Wasserstoff, A C=O und B C-OH bedeuten und die gestrichelte Linie die obengenannte Bedeutung hat, und pharmazeutisch verwendbare Salze solcher Verbindungen.

Im folgenden werden die erfindungsgemässen Verbindungen mit Saframycin Mx 1 ($R^1$ = OH) und Saframycin Mx 2 ($R^1$ = H) bezeichnet. Ohne Zusatz bedeuten diese Bezeichnungen Verbindungen der Formel I, in denen A C=O und B C-OH ist. Mit dem Zusatz "BC" (Bis-chinon) werden diese Bezeichnungen für Verbindungen verwendet, in denen A C=O und B C=O bedeuten. Saframycin Mx 1 BHC (Bis-hydrochinon) ist die Verbindung der Formel I, worin $R^1$ OH, $R^2$ Wasserstoff. A C-OH und B C-OH sind. Mit dem Zusatz "Acyl" werden entsprechende Verbindungen bezeichnet, in denen $R^2$ Acyl bedeutet.

Erfindungsgemässe Salze von Verbindungen der Formel I sind in erster Linie pharmazeutisch verwendbare, nicht-toxische Säureadditionssalze. Beispiele für Säureadditionssalze mit nicht-toxischen, physiologisch gut verträglichen Säuren sind Salze mit anorganischen Säuren, z.B. Salzsäure, Schwefelsäure oder Phosphorsäure, oder mit organischen Carbon-, Sulfon- oder Sulfonsäuren, z.B. Ameisensäure, Essigsäure, Propionsäure, Glykolsäure, Bernsteinsäure, Maleinsäure, Hydroxymaleinsäure, Methylmaleinsäure, Fumarsäure, Aepfelsäure, Weinsäure, Zitronensäure, Benzoesäure, Zimtsäure, Mandelsäure, Salicylsäure, 4-Aminosalicylsäure, 2-Phenoxybenzoesäure, 2-Acetoxybenzoesäure, Embonsäure, Nicotinsäure oder Isonicotinsäure, ferner

Aminosäure, z.B. α-Aminosäuren, sowie Methansulfonsäure, Ethansulfonsäure, 2-Hydroxyethansulfonsäure, Ethan-1,2-disulfonsäure, Benzolsulfonsäure, 4-Methylbenzolsulfonsäure oder Naphthalin-2-sulfonsäure, oder mit anderen sauren organischen Verbindungen, wie Ascorbinsäure. Zur Isolierung oder Reinigung können auch pharamzeutisch ungeeignete Salze Verwendung finden.

Die erfindungsgemässen Verbindungen der Formel I und ihre pharmazeutisch verwendbaren Salze weisen wertvolle pharmakologische Eigenschaften auf. Beispielsweise sind Verbindungen der Formel I, in denen A C=O bedeutet, in niedriger Konzentration von 0,004 µg/ml bis 0,3 µg/ml gegen verschiedene Keime wirksam, z.B. gegen Staphyhlococcus aureus, Bacillus subtilis, Micrococcus luteus und Escherichia coli. Neben antibiotischen Eigenschaften weisen die Verbindungen auch tumorhemmende Eigenschaften auf, beispielsweise gegen die experimentellen Tumoren Leukämie L 1210/S2, menschliches Lungenkarzinom MBA 9812, Colon Adenokarzinom 26 und Reticulum Zellsarkom M5076.

Werden Mäuse mit Leukämie L 1210/S2 mit i.p. Injektionen von 2,5 oder 5 mg/kg Saframycin Mx 2 an vier aufeinanderfolgenden Tagen behandelt, so verlängert sich ihre Lebensdauer signifikant um den Faktor 1,6 oder mehr. Bei der Behandlung mit vier Injektionen von 0,625, 1,25 oder 2,5 mg/kg Saframycin Mx 1 verlängert sich die Lebensdauer um den Faktor 1,7 oder mehr. Saframycin Mx 2 in 7 Tagesdosen zu 2,5 oder 5,0 mg/kg i.p. reduziert das Gewicht von experimentell in Balb/c Nackt-Mäuse implantiertem humanem Lungenkarzinom MBA 9812 im Vergleich zu unbehandelten Tieren signifikant auf 62 % oder weniger. Saframycin Mx 1 in 10 Dosen zu 1,25 mg/kg i.p. reduziert das Gewicht von Colon Adenokarzinom 26 im Vergleich zu unbehandelten Tieren auf 76 %. Nach 17 Dosen zu 1,25 mg/kg i.p. von Saframycin Mx 1 wird das Gewicht von Reticulum Zellsarkom M5076 im Vergleich zu unbehandelten Tieren gar auf 33 % reduziert. Dabei weisen Saframycin Mx 1 und Mx 2 in Mäusen eine maximal tolerierbare einmalige Dosis von 12,5 mg/kg bzw von über 25 mg/kg auf.

Verfahren zur Herstellung von Verbindungen der Formel I sind ebenfalls Gegenstand der Erfindung. Verbindungen der Formel I werden hergestellt, indem man den Stamm Mx x48 der Species Myxococcus xanthus oder eine von diesem abgeleitete, die Verbindungen der Formel I produzierende Mutante in einer Kultur enthaltend Kohlenstoff- und Stickstoffverbindungen und essentielle anorganische Salze in leicht assimilierbarer Form bei einer Temperatur zwischen 15°C und 40°C und einem pH-Wert zwischen 5 und 9 unter aeroben Bedingungen züchtet, die gebildeten Verbindungen der Formel I isoliert und, wenn erwünscht, eine erhältliche Verbindung der Formel I in eine andere Verbindung der Formel I und/oder eine erhältliche Verbindung in ein Salz und/oder ein erhältliches Salz in die freie Verbindung oder in ein anderes Salz überführt.

Der Mikroorganismus Myxococcus xanthus, Stamm Mx x48, ist ebenfalls Gegenstand der Erfindung. Er wurde aus einer Bodenprobe der Oase Gabès, Tunesien isoliert und ist bei der National Collection of Industrial and Marine Bacteria, Aberdeen, Schottland, unter der Nummer NCIB 12 268 hinterlegt.

Der Stamm Mx x48 kann spontan natürliche Mutanten bilden, die Verbindungen der Formel I produzieren. Künstliche Mutanten kann man beispielsweise chemisch, z.B. durch Behandlung mit alkylierenden oder nitrosierenden Verbindungen, z.B. N-Methyl-N'-nitro-N-nitrosoguanidin oder mit Alkalinitrit, wie Natriumnitrit, oder durch Bestrahlung, z.B. mit energiereicher Strahlung, wie Ultraviolett-, Röntgen- oder radioaktiver Strahlung, erzeugen. Solche Mutanten des Stammes Mx x48, die Verbindungen der Formel I produzieren, gehören ebenfalls zum Gegenstand der Erfindung.

Im Verfahren zur Herstellung der Verbindungen der Formel I wird der Mikroorganismus Myxococcus xanthus Stamm Mx x48 unter geeigneten Bedingungen kultiviert.

Die zur Züchtung verwendbare Kultur muss eine Kohlenstoff- und Stickstoffquelle sowie essentielle anorganische Salze enthalten. Als Kohlenstoffquelle und Stickstoffquelle geeignet sind Aminosäuren, Peptide und Proteine sowie deren Abbauprodukte wie Pepton oder Trypton, ferner Fleischextrakte, Getreidemehl, z.B. von Mais oder Weizen, Bohnen, insbesondere Sojabohnen, Fischmehl, Samen, beispielsweise der Baumwollpflanze, Destillationsrückstände der Alkoholherstellung, Hefeextrakte usw. Als essentielle anorganische Salze kann die Nährlösung beispielsweise Chloride, Carbonate, Sulfate, Phosphate von Alkali- oder Erdalkalimetallen, z.B. Natrium, Kalium, Magnesium und Calcium, ferner Salze von Eisen, Zink, Mangan, Molybdän und Kupfer enthalten. Die Züchtung erfolgt bevorzugt in Flüssigkulturen, insbesondere wässrigen Kulturen.

Als flüssiges Kulturmedium eignet sich insbesondere das Medium MD1 (Pepton aus Casein, tryptisch verdaut, 0,3 %; $CaCl_2 \cdot 2H_2O$ 0,05 %; $MgSO_4 \cdot 7H_2O$ 0,2 %). Andere geeignete flüssige Kulturmedien bestehen beispielsweise aus Pepton aus Casein 0,05 %, $CaCl_2 \cdot 2H_2O$ 0,05 %, $MgSO_4 \cdot 7H_2O$ 0,2 %, dem wahlweise 0,5 % Einzellerprotein, Maisquellwasser, Hefeextrakt, Aminosäurehydrolysat aus Casein oder Protein aus Fischmehl zugegeben wird.

Die Züchtung erfolgt bei Temperaturen zwischen 15°C und 40°C, bevorzugt bei Temperaturen zwischen 25°C und 35°C, z.B. um 30°C. Geeignete pH-Werte liegen zwischen pH 5 und pH 9, bevorzugt zwischen pH 6,5 und pH 8,5.

Die Züchtung kann ansatzweise, z.B. durch ein- oder mehrmalige Zugabe von Nährlösung, oder kontinuierlich durch dauernde Zugabe von Nährlösung erfolgen. Vorzugsweise züchtet man in mehreren Stufen, indem man eine Vorkultur, z.B. in einem der genannten Kulturmedien, herstellt (Inoculum), welche man anschliessend nach ca. ein- bis zweitägiger Fermentation in eine grössere Kultur, beispielsweise in einem Verdünnungsverhältnis zwischen 1:10 und 1:500, überimpft. Diese Kultur kann wiederum nach ca. zwei- bis dreitägiger Fermentation in eine noch grössere Hauptkultur, beisielsweise in einem Verdünnungsverhältnis von 1:10 bis 1:100, überimpft werden.

Die erste Vorkultur kann man auch durch mehrtägiges Wachstum des Stammes Mx x48 auf festem oder flüssigem Nährboden, z.B. Agar enthaltend eines der genannten flüssigen Kulturmedien, erhalten. Geeignet als Nährboden sind beispielsweise Agar 1,5 % $CaCl_2 \cdot 2H_2O$ 0,1 % enthaltend Backhefe 0,5 % oder Pepton aus Casein 0,3 % und Hefeextrakt 0,1 %.

Man kann den Fermentationsverlauf durch Probenentnahme analytisch während der Fermentation verfolgen, z.B. durch Messung des pH-Wertes der Kultur, welcher während der Fermentation von ca. pH 7,2 auf etwa pH 8,0 ansteigt, der optischen Dichte, welche ein Mass für das Wachstum des Stammes ist, gravimetrisch anhand des Trockengewichts der gebildeten Zellmasse, durch Dünnschichtchromatographie, durch Hochdruck-Flüssigchromatographie an Umkehrphasen oder durch Bestimmung der antibiotischen Aktivität der im Kulturfiltrat enthaltenen Komponenten. Beispielsweise kann man zellfreien Kulturüberstand unverdünnt oder in verdünnter Form in einem Agardiffusionstest gegen Staphylococcus aureus prüfen.

Die Isolierung der erfindungsgemässen Verbindungen der Formel I aus der Kulturbrühe erfolgt nach an sich bekannten Methoden unter Berücksichtigung ihrer chemischen, physikalischen und biologischen Eigenschaften. Zum Bestimmen der Konzentration der erfindungsgemässen Verbindungen in den einzelnen Isolierungsstufen kann die Dünnschichtchromatographie, z.B. auf Silicagel mit Methylenchlorid/Methanol, die Hochdruck-Flüssigchromatographie, z.B. an Umkehrphasen-Silicagel, und/oder die Aktivität gegen Staphylococcus aureus im Agardiffusionstest verwendet werden.

Für die Isolierung der erfindungsgemässen Verbindungen aus der rohen Fementationsbrühe wird diese vorzugsweise mehrere Stunden mit makroporösen, nicht-ionischen Adsorberharzen, z.B. synthetischen Harzen mit aromatischem Grundgerüst, beispielsweise Harzen auf Polystyrolbasis, z.B. Styrol-Divinylbenzol-Copolymeren, gerührt. Solche Harze lassen sich durch verschiedene gebräuchlice statistische Kenngrössen, z.B. Porenvolumen, spezifische Oberfläche, durchschnittliche Porendurchmesser, häufigster Porendurchmesser, Porengrössenverteilung, Korngrössenverteilung und dergleichen charakterisieren. Geeignete Adsorberharze haben ein Porenvolumen von ca. 0,5 bis ca. 4,5 ml/g, eine spezifische Oberfläche von ca. 100-1000 $m^2/g$ und einen durchschnittlichen Porendurchmesser von ca. 4 bis ca. 130 nm und sind beispielsweise unter den Handelsnamen AMBERLITE® XAD-1, XAD-2, XAD-4, XAD-1180 und ER-180 der Fa. Röhm & Haas, DIAION® HP-10, HP-20, HP-21, HP-30, HP-40, HP-50 der Fa. Mitsubishi, DUOLITE® S-861, S-862, S-863 und ES 866 der Fa. Dia-Prosim, IMAC® Syn 46 und Syn 72 der Firma Akzo Chemie, KASTEL® S-111, S-112, S-114 der Fa. Montedison, LEWATIT® OC.1031 der Fa. Bayer und RELITE® ADS der Fa. Resindion, erhältlich.

Nach Abtrennung der Fermentationsbrühe vom Adsorberharz, z.B. durch Sieben, wird dieses mit Wasser gewaschen und anschliessend mit einem organischen Lösungsmittel oder einem Gemisch von Wasser mit einem organischen Lösungsmittel, welches gegenüber dem verwendeten Adsorberharz inert ist, z.B. mit Isopropanol, eluiert. Die Eluate werden gewünschtenfalls mit einer organischen oder anorganischen Säure behandelt und im Vakuum eingeengt.

Es ist auch möglich, das Gemisch der Fermentation auf klassiche Art und Weise aufzuarbeiten. Die Kulturbrühe wird dazu von der Zellmasse auf übliche Weise, z.B. durch Filtration oder Zentrifugation, abgetrennt und das Kulturfiltrat mit einem mit Wasser nur wenig oder nicht mischbaren organischen Lösungsmittel, z.B. Methylenchlorid, Chloroform oder bevorzugt Essigsäureethylester, extrahiert. Nach Eindampfen im Vakuum erhält man einen Rohextrakt. Bei Verteilung des Rohextrakts im Zweiphasensystem bestehend aus zwei nicht miteinander mischbaren organischen Lösungmitteln, z.B. Methanol-Heptan, reichern sich die Fermentationsprodukte, insbesondere die erfindungsgemäßen Verbindungen der Formel I, in der polaren Phase an.

Die rohen Extrakte werden vorzugsweise mit chromatographischen Methoden gereinigt, beispielsweise durch Chromatographie an einem Ionenaustauscher mit schwach sauren funktionellen Gruppen, z.B. an Ionenaustauscher mit Carboxy-Gruppen, Adsorptions- bzw. Verteilungschromatographie und/oder Hochdruck-Flüssigchromatographie an unpolaren Oberflächen, z.B. an Silicagel enthaltend langkettige Alkylgruppen ("reversed phase") oder an Agarose mit Alkyl- oder Phenylgruppen ("hydrophobic interaction"). Auch andere chromatographische Methoden kommen in Betracht, beispielsweise Affinitätschromatographie, "fast pressure liquid chromatography" und dergleichen, ferner Gegenstromverteilung, z.B. "droplet counter-current chromatography" oder "rotational locular counter-current chromatography".

Geeignetes Trägermaterial für die Ionenaustauscher-Chromatographie sind organische Polymere, z.B. quervernetzte Agarose, Dextran, Polyacrylamid, Styrol-Divinylbenzol-Copolymer oder Cellulose. Vorzugsweise trägt dieses Material schwach saure funktionelle Gruppen, z.B. Carboxy-Gruppen. Als Inonenaustauscher bevorzugt sind Träger aus quervernetztem Dextran enthaltend Carboxylalkyl-Reste, z.B. Carboxymethyl-Reste, wie sie beispielsweise unter dem Handelsnamen CM-Sephadex® erhältlich sind.

Das Ionenaustauschermaterial mit Carboxygruppen wird in geeigneten wässrigen Pufferlösungen, z.B. Pufferlösungen zwischen pH 5 und pH 7, z.B. Phosphatpuffer pH 5, vorbehandelt. Dann werden die Rohextrakte enthaltend die erfindungsgemässen Verbindungen zugegeben, beispielsweise indem man diese Extrakte auf eine Säule enthaltend den vorbehandelten Ionenaustauscher aufträgt. Ungebundene Substanzen und Verunreinigungen werden mit Pufferlösung aus der Ionenaustauschersäule gegebenenfalls unter Druck ausgewaschen und die erwünschten Saframycine durch Pufferlösungen enthaltend eine zunehmende Salz-Konzentration, z.B. Phosphatpuffer pH 5 enthaltend zunehmende Konzentrationen an Natriumchlorid, aus der Säule eluiert. Die beschriebene Ionenaustauscherchromatographie kann gewünschtenfalls auch auf für Hochdruck-Flüssigchromatographie geeigneten Säulen durchgeführt werden.

Geeignetes Trägermaterial für Adsorptions- und Verteilungschromatographie sind beispielsweise organische Polymere, z.B. quervernetzte Agarose, Dextran oder Cellulose. Bevorzugt für die Reinigung der Saframycine durch Adsorptions- und Verteilungschromatographie sind quervernetzte Dextrane, die geeignete funktionelle Gruppen tragen, z.B. Hydroxylalkyl-Gruppen. Besonders bevorzugt ist ein quervernetztes Dextran mit Hydroxypropylgruppen, das unter dem Handelsnamen Sephadex® LH-20 erhältlich ist.

Bevorzugt werden die durch Ionenaustauscher-Chromatographie vorgereinigten Extrakte auf eine Säule enthaltend das Trägermaterial mit Hydroxylpropylgruppen aufgetragen und mit Gemischen organischer Lösungsmittel gleichbleibender oder graduell wechselnder Zusammensetzung eluiert. Vorzugsweise werden Methanol oder Mischungen von Methanol mit einem wenig polaren weiteren organischen Lösungsmittel, z.B. mit Methylenchlorid, unter Zusatz einer organischen Säure, beispielsweise Ameisen- oder Essigsäure, z.B. ein Methanol/Methylenchlorid-Gemisch enthaltend ca. 0,1 % Essigsäure, verwendet. Wie die Ionenaustauscherchromatographie kann auch die Adsorptions- und Verteilungschromatographie an für Hochdruck-Flüssigchromatographie geeigneten Säulen durchgeführt werden.

Zur Trennung der Verbindungen der Formel I werden durch Ionenaustauscher- und/oder Adsorptionschromatographie vorgereinigte Extrakte vorzugsweise einer Hochdruck-Flüssigchromatographie an unpolaren Oberflächen, sogenannte "reversed phase" (Umkehrphasen)-Chromatographie, unterworfen. Bevorzugt wird dazu ein Trägermaterial auf Silicagel-Grundlage verwendet, das langkettige Alkylgruppen trägt, z.B. Alkylgruppen von 14 bis 22 C-Atomen, z.B. 18 C-Atomen. Solche Trägermaterialien sind beispielsweise unter den Handelsnamen HD-Sil® 18-10-60, Nucleosil® C-18, Bio-Sil® ODS-10 oder Hi-Pore® RP-318 erhältlich. Das zu trennende Saframycin-Gemisch wird auf eine Umkehrphase-Hockdruck-Flüssigchromatographie-Säule aufgegeben und in wässrig-organischer Lösung enthaltend Säure entwickelt, beispielsweise in Mischungen von Wasser mit Methanol, Acetonitril oder Tetrahydrofuran enthaltend eine organische Sulfonsäure, z.B. Heptansulfonsäure, Trifluoressigsäure oder eine anorganische schwache Säure, z.B. Dihydrogenphosphat. Bevorzugt sind Methanol/Wasser-Mischungen mit Zusatz von Phosphatpuffer pH 6,5.

Die Reinigung der Rohextrakte kann auch durch GegenstromverteilungsChromatographie erfolgen, beispielsweise durch "droplet counter-current chromatography" oder bevorzugt durch "rotational locular counter-current chromatography". Als wässrige Phase sind schwach saure Pufferlösungen geeignet, z.B. Pufferlösungen zwischen pH 4 und pH 7, bevorzugt Phosphatpuffer pH 5. Als organische Phase verwendet man beispielsweise Chlorkohlenwasserstoff, z.B. Chloroform oder Methylenchlorid, dem gewünschtenfalls ein Niederalkanol beigemischt ist, z.B. n-Butanol, iso- oder n-Propanol, auch Methanol, vorzugsweise eine Mischung von Methylenchlorid und iso-Propanol.

Die Umwandlung einer Verbindung der Formel I in eine andere Verbindung der Formel I geschieht mit an sich bekannten Methoden, beispielsweise durch Oxidation, durch Reduktion oder durch Acylierung.

Umwandlungen von Hydrochinonen (A und/oder B mit der Bedeutung C-OH) in ensprechende Chinone (A und/oder B mit der Bedeutung C=O) durch Oxidation und Umwandlungen der Chinone in Hydrochinone durch Reduktion können beispielsweise analog den in "Methoden der Organischen Chemie (Houben-Weyl)", 4. Auflage, Band VII/3a, Thieme Verlag Stuttgart 1977, beschriebenen Verfahren durchgeführt werden. Geeignete milde Oxidationsmittel sind beispielsweise Metallsalze und Metalloxide, beispielsweise Silber(I)-oxid in Diethylether, Benzol oder Toluol in Gegenwart eines Trockenmittels, z.B. Natriumsulfat, Eisen(III)salze, wie Eisen(III)chlorid in Wasser oder wässrigem Ethanol, Kupfersalze oder Thalliumsalze, z.B. Thallium(III)trifluoracetat. Ebenfalls geeignet als Oxidationsmittel ist Luftsauerstoff in neutraler Lösung, z.B. in Pufferlösungen pH 7 bis pH 8. Geeignete Reduktionsmittel sind beispielsweise Wasserstoff in Gegenwart heterogener Katalysatoren, z.B. Platinoxid oder Palladium auf Kohle, oder homogener Katalysatoren, z.B Tris(triphenylphosphin)rhodium(I)chlorid, ferner Metallhydride, z.B. Borhydride, wie Natriumborhydrid oder Aluminiumhydride, wie Lithiumaluminiumhydrid, reduzierende Salze, z.B. Natriumdithionit, ferner auch Licht in Gegenwart eines Wasserstoff-Donators, beispielsweise gewöhnliches Tageslicht und Methanol. Umwandlungen der Chinone und Hydrochinone ineinander können auch durch elektrochemische Methoden, beispielsweise Elektrolyse in schwach sauren, salzhaltigen Lösungen, z.B. Pufferlösungen pH 4 bis pH 6, vorgenommen werden.

Die Acylierung einer Verbindung der Formel I, worin $R^2$ Wasserstoff bedeutet, erfolgt nach an sich bekannten Methoden, beispielsweise mit einer Säure der Formel $R^2$-OH oder einem reaktiven funktionellen Derivat dieser Säure.

Wenn eine freie Carbonsäure für die Acylierung verwendet wird, wird die Reaktion üblicherweise in Gegenwart eines geeigneten Kondensationsmittels durch geführt, z.B. eines Carbodiimids, beispielsweise Dicyclohexyl- oder vorzugsweise N-Ethyl-N′-3-dimethylaminopropyl-carbodiimid. Die Kondensation erfolgt in einem wässrig-organischen Lösungsmittelgemisch oder einem wässrigen Puffer nahe dem Neutralpunkt, z.B. zwischen pH 6 und 8, gegebenenfalls unter Kühlung oder Erwärmung und in einer inerten Gas-Atmosphäre, z.B. unter Stickstoff.

Ein reaktives funktionelles Derivat einer Carbonsäure oder eines Kohlensäurehalbesters ist ein entsprechendes Anhydrid, beispielsweise ein symmetrisches Anhydrid, z.B. Essigsäureanhydrid, ein gemischtes Anhydrid der Säure $R^2$-OH mit einer anorganischen oder organischen Säure, z.B. mit Halogenwasserstoffsäure, wie Salzsäure oder Bromwasserstoffsäure, also beispielsweise ein Carbonsäurechlorid oder -bromid beziehungsweise ein Chlorameisensäureester, oder ein gemischtes Anhydrid einer Carbonsäure mit einem Halbester der Kohlensäure, z.B. dem Ethyl- oder Isobutyl-halbester der Kohlensäure. Ein weiteres reaktives funktionelles Derivat einer Carbonsäure oder eines Kohlensäurehalbesters ist

beispielsweise ein aktivierter Ester, z.B. ein N-Hydroxyester, wie der Ester mit N-Hydroxypiperidin, N-Hydroxysuccinimid, N-Hydroxyphthalimid oder 1-Hydroxybenztriazol.

Die Acylierungsreaktionen mit reaktiven funktionellen Derivaten von $R^2$-OH werden vorzugsweise in wässrig-organischen homogenen oder zweiphasigen Lösungsmittelgemischen durchgeführt, wenn erwünscht unter Kühlung oder leichter Erwärmung, z.B. im Temperaturbereich von ungefähr 0°C bis ungefähr 40°C, vorzugsweise um Raumtemperatur, und gegebenenfalls in einer Inertgas-Atmosphäre, z.B. unter Stickstoff. Dabei wird ein Säureakzeptor zugegeben, beispielsweise eine geeignete organische Base, z.B. ein Amin, z.B. Trimethylamin, Triethylamin, N-Methylmorpholin, 1,5-Diazabicyclo[4.3.0.]non-5-en, 1,8-Diazabicyclo[5.4.0]undec-7-en oder Pyridin, oder eine anorganische Base, beispielsweise ein Alkalimetall- oder Erdalkalimetallhydroxyd, z.B. Natrium-, Kalium- oder Calcium-hydroxid, ein Alkalimetall- oder Erdalkalimetallcarbonat, z.B. Natriumcarbonat, Natriumhydrogencarbonat oder Calciumcarbonat, oder ein Alkalimetallphosphat, z.B. Natrium- oder Kalium-phosphat oder -hydrogenphosphat. Vorzugsweise wird die Acylierungsreaktion in einem wässrigen Puffer nahe dem Neutralpunkt, z.B. zwischen pH 6 und pH 8, durchgeführt, wobei die Pufferbase die Rolle des Säureakzeptors übernimmt. Um die Acylierung einer phenolischen Hydroxygruppe A und/oder B und gegebenenfalls einer Hydroxygruppe $R^1$ zurückzudrängen, wird dem wässrig-organischen Lösungsmittelgemisch beispielsweise ein Alkohol, z.B. Methanol oder Ethanol, zugegeben.

Salze von Verbindungen der Formel I können in an sich bekannter Weise hergestellt werden, beispielsweise durch Umsetzen der freien Verbindung mit vorzugsweise stöchiometrischen Mengen oder einem kleinen Ueberschuss der ein Säureadditionssalz bildenden Säure.

Salze können in üblicher Weise in die freien Verbindungen übergeführt werden, beispielsweise durch Behandeln mit einer äquimolaren Menge einer freien Base, z.B. eines Hydroxids, wie Alkalihydroxids, z.B. Lithium-, Kalium- oder Natriumhydroxids, Erdalkalihydroxids, z.B. Calciumhydroxids oder Magnesiumhydroxids, oder Ammoniumhydroxids, z.B. unsubstituierten Ammoniumhydroxids oder Benzyltrimethylammoniumhydroxids, oder eines organischen tertiären Amins, z.B. Triethylamin. Dabei ist jedoch zu beachten, dass die freie Verbindung der Formel I nur beschränkt haltbar ist und zur Lagerung in ein Säureadditionssalz übergeführt werden muss.

Salze von Verbindungen der Formel I werden in an sich bekannter Weise in andere Salze überführt. Vorzugsweise geschieht die Umwandlung von Salzen in andere Salze mit Ionenaustauschern, die mit dem gewünschten Anion beladen sind, oder durch Adsorptionschromatographie in einem Lösungsmittel, das die Säure des gewünschten Säureadditionssalzes im Ueberschuss enthält.

Die Erfindung betrifft auch diejenigen Ausführungsformen des Verfahren, bei der man von einem auf irgendeiner Stufe der Isolierung erhältlichen Extrakt ausgeht und die abschliessenden Schritte durchführt, das Verfahren auf irgendeiner Stufe abbricht und/oder eine nach dem erfindungsgemässen Verfahren erhältliche Verbindung in situ weiterverarbeitet.

Die Verbindungen der vorliegenden Erfindung und ihre pharmazeutisch verwendbaren Salze können z.B. zur Herstellung von pharmazeutischen Präparaten verwendet werden, welche eine wirksame Menge des Wirkstoffs vorzugsweise im Gemisch mit einer signifikanten Menge von anorganischen oder organischen, festen oder flüssigen, pharmazeutisch verwendbaren Trägerstoffen enthalten. Pharmazeutische Präparate, ihre Herstellung und Verwendung sind ebenfalls Gegenstand der vorliegenden Erfindung.

Die erfindungsgemässen pharmazeutischen Präparate eignen sich zur oralen oder bevorzugt parenteralen, z.B. intravenösen, intramusculären oder topischen Verabreichung.

Beispielsweise verwendet man die Wirkstoffe der Formel I der vorliegenden Erfindung in Form von injizierbaren, z.B. intravenös, intramuskulär, intradermal oder subcutan verabreichbaren Präparaten oder Infusionslösungen. Solche Lösungen sind vorzugsweise isotonische wässrige Lösungen oder Suspensionen, welche z.B. aus lyophilisierten Präparaten, welche den reinen Wirkstoff oder den Wirkstoff zusammen mit einem Trägermaterial, z.B. Dextran, Mannit oder Albumin enthalten, vor Gebrauch hergestellt werden können. Pharmazeutische Präparate zur oralen Anwendung sind gegebenenfalls sterilisiert und können Hilfsstoffe, z.B. Konservier-, Stabilisier-, Netz- und/oder Emulgiermittel, Löslichkeitsvermittler, Salze zur Regulierung des osmotischen Druckes und/oder Puffer enthalten. Geeignet sind auch entsprechende ölige Injektionssuspensionen, wobei man als Träger lipophile Lösungsmittel oder Vehikel, beispielsweise fette Oele, z.B. Sesamöl, Triglyceride oder synthetische Fettsäureester, z.B. Ethyloleat, verwendet. Die injizierbaren pharmazeutischen Präparate, die, wenn erwünscht, weitere pharmakologisch wertvolle Stoffe, z.B. andere Wirkstoffe, enthalten können, enthalten etwa 0,1 % bis 100%, insbesondere etwa 1 % bis 50 %, im Falle von Lyophilisaten bis zu 100 %, des Wirkstoffes.

Geeignete Trägerstoffe für orale Präparate, z.B. Dragées, Tabletten oder Lacktabletten, sind insbesondere Füllstoffe, wie Zucker, z.B. Lactose, Saccharose, Mannit oder Sorbit, Cellulosepräparate und/oder Calciumphosphate, z.B. Tri-calciumphosphat oder Calciumhydrogenphosphat, Bindemittel, wie Stärkekleister auf der Basis z.B. von Mais-, Weizen-, Reis- oder Kartoffelstärke, Gelatine, Methylcellulose, Hydroxypropylmethylcellulose, Hydroxypropylcellulose und/oder Polyvinylpyrrolidon, und/oder, wenn erwünscht, Sprengmittel, wie die genannten Stärken, ferner Carboxymethylstärke, quervernetztes Polyvinylpyrrolidon, Agar, Alginsäure oder ein Salz davon, wie Natriumalginat oder niedermolekulare Carboxymethylcellulose. Hilfsmittel sind in erster Linie Fliessregulier- und Schmiermittel, z.B. Kieselsäure, Talk, Stearinsäure oder Salze davon, wie Magnesium- oder Calciumstearat, und/oder Polyethylenglykol.

Dragéekerne werden mit geeigneten, gegebenenfalls Magensaftresistenten Ueberzügen versehen. Weitere

oral anwendbare pharmazeutische Präparate sind Steckkapseln aus Gelatine sowie weiche, geschlossene Kapseln aus Gelatine und einem Weichmacher, wie Glycerin oder Sorbit. Als rektal anwendbare pharmazeutische Präparate kommen z.B. Suppositorien in Betracht, welche aus einer Kombination des Wirkstoffs mit einer Suppositoriengrundmasse bestehen. Oral und rektal anwendbare Präparate enthalten etwa 0,1 % bis etwa 50 %, insbesondere etwa 1 % bis etwa 10 %, des Wirkstoffes und, wenn erwünscht, weitere pharmakologisch wertvolle Verbindungen.

Pharmazeutische Präparate für topische Anwendungen sind insbesondere Crêmen, Gele, Salben, Pasten, Schäume, Tinkturen und Lösungen, die von etwa 0,05 % bis etwa 10 %, insbesondere von etwa 0,5 % bis etwa 5 % des Wirkstoffes enthalten.

Die pharmazeutischen Präparate werden in an sich bekannter Weise, z.B. mittels konventioneller, in Pharmakopöen beschriebener Lösungs-, Suspendier-, Misch- oder Lyophilisierungsverfahren, herstellt.

Die Erfindung betrifft ebenfalls die Verwendung der Verbindungen der Formel I oder von Salzen davon als Heilmittel, beispielsweise in Form von pharmazeutischen Präparaten, zur Behandlung von bakteriellen Infektionen durch gram-positive Keime und zur Behandlung von Tumoren, insbesondere Lungen- und Magen/Darm-Tumoren und Leukämie, bei Warmblütern, z.B. Menschen und Säugetieren, durch enterale, z.B. orale, oder bevorzugt parenterale Applikation von therapeutisch wirksamen Dosen.

Die zur Anwendung gelangenden täglichen Dosen betragen je nach Spezies, Alter, individuellem Zustand, Schwere der Erkrankung und Applikationsweise entsprechend der Beurteilung des behandelnden Arztes zwischen etwa 0,01 mg und etwa 10 mg, insbesondere zwischen etwa 1,0 mg und etwa 1 mg pro kg Körpergewicht.

Die nachfolgenden Beispiele illustrieren die Erfindung, schränken deren Umfang jedoch in keiner Weise ein.

Beispiel 1: Bakterium Myxococcus xanthus Mx x48

a) Herknuft und Zugänglichkeit des Produktionsstamms: Das Bakterium Myxococcus xanthus Stamm Mx x48, Familie Myxococcaceae, Ordnung Myxobacterales, wurde im Mai 1980 aus einer Bodenprobe der Oase Gabès, Tunesien, isoliert. Der Stamm Mx x48 ist bei der National Collection of Industrial and Marine Bacteria, Aberdeen, Schottland, am 26. Mai 1986 unter der Nummer NCIB 12 268 für Patentzwecke nach dem Budapester Vertrag hinterlegt worden.

b) Beschriebung des Produktionsstamms: Vegetative Stäbchen, zylindrisch mit kurz verjüngten Enden, zigarrenförmig, 0,8 x 3,5-5 μm, bewegen sich auf Oberflächen kriechend-gleitend. Auf geeigneten Medien, z.B. VY/2-Agar (s. unten), bildet der Organismus "Fruchtkörper" von 50-150 μm Durchmesser in Form von intensiv rotorangen bis blass grauen weichschleimigen Tropfen, Pusteln oder Köpfchen auf der Agaroberfläche. In deren Inneren finden sich kugelige, stark lichtbrechende Myxosporen von 1,8-1,9 μm Durchmesser. Die Kolonien tendieren dazu, sich hauchartig als "Schwärme" auf der Agaroberfläche auszubreiten und können bis zu mehreren cm gross werden. Auf manchen Medien sind sie intensiv grünlich-gelb bis orange gefärbt.

c) Kulturbedingungen: Der Organismus wächst gut auf Peptonagar, z.B. CY-Agar (Casitone [Difco] 0,3 %, Hefeextrakt [Difco] 0,1 %, $CaCl_2 \cdot 2H_2O$ 0,1 %, Agar 1,5 %, pH 7,2) oder auf Hefeagar, z.B. VY/2-Agar (Backhefe 0,5 % bezogen auf Frischgewicht, $CaCl_2 \cdot 2H_2O$ 0,1 %, Agar 1,5 %, pH 7,2). In Flüssigmedien wächst Mx x48 in homogener Zellsuspension sowohl in Schüttelkolben (bei etwa 160 upm) als auch in Bioreaktoren. Als Kulturmedium ist z.B. geeignet MDI 1.m. (Pepton aus Casein, tryptisch verdaut [Merck, Darmstadt] 0,3 %, $MgSO_4 \cdot 7H_2O$ 0,2 %, $CaCl_2 \cdot 2H_2O$ 0,05 %, pH 7,2). Mx x48 ist streng aerob. Alle Kulturen werden bei 30° C gehalten. Die Generationszeit in MDI 1.m. liegt bei etwa 6,5 Stunden ($\mu$ = 0,15 pro Stunde). Die maximal erreichte optische Dichte (623 nm, 1 cm Weglänge) liegt bei 1,3 bis 1,5. Die Konzentration des Peptons aus Casein kann auch erhöht werden, z.B. auf 0,5 oder 1 %. Die Generationszeit ändert sich dabei nicht, dagegen erhöht sich die optische Dichte auf ca. 2 bzw. 3,5. Die Antibiotikumproduktion wird bei steigender Pepton-Konzentraton aber geringer im Vergleich zur Produktion in MDI 1.m. Als Kulturmedien, in denen der Stamm unter Antibiotikumbildung wächst, sind ferner folgende Medien geeignet: 0,05 % Casitone, 0,2 % $MgSO_4 \cdot 7H_2O$ und 0,05 % $CaCl_2 \cdot 2H_2O$, pH 7,2, enthaltend entweder 0,5 % Probion S (Einzellerprotein der Fa. Hoechst) oder 0,5 % Maisquellwasser oder 0,5 % Protaminal (Fischproteinkonzentrat der Fa. Asta) oder 0,5 % Hefeextrakt oder 0,3 % Casamino acids (Difco).

d) Konservierungsmöglichkeiten: 1. Durch Einfrieren vegetativer Zellen von Platten- oder Flüssigkulturen in Pepton-Lösung bei -80° C (mehrere Jahre lebensfähig). 2. Durch Trocknen von Fruchtkörpern auf Filterpapier (mehrere Jahre bei Zimmertemperatur lebensfähig). 3. Durch Trocknen von Fruchtkörpern in Milch, Lagerung in zugeschmolzenen Ampullen unter Stickstoff bei Zimmertemperatur (mehrere Jahre lebensfähig). 4. Durch Suspendieren vegetativer Zellen von Platten oder Flüssigkulturen in Peptonlösung und Einfrieren in flüssigem Stickstoff (mehrere Jahre lebensfähig).

Beispiel 2: Herstellung von Zellüberständen mit antibiotischer Wirkung

a) Produktionsbedingungen: In Schüttelkolben ist die Aktivität gegen Staph. aureus am Ende der logarithmischen Wachstumsphase (OD 0,9-1) mit Hilfe des Agardiffusionstestes nachzuweisen. Die höchste Aktivität ist in der frühen stationären Phase erreicht. Der pH in der Kultur liegt dann bei etwa 8 und steigt bei weiterer Inkubation auf etwa 8,5, ohne dass die optische Dichte weitere zunimmt. Vielmehr kommt es in der späten stationären Phase zur Verklumpung der Zellen und zur Zell-Lyse. Die antibiotische Aktivität nimmt dabei wieder ab.

b) Produktion im Fermenter:

1. Vorkultur: 50 ml MDI 1.m. werden mit 0,5 ml angeimpft und bei 30°C und 160 upm über 2 Tage gehalten.

2. Vorfermentation: Ein 25 l-Bioreaktor der Firma Braun, Melsungen, mit Blattrührsystem wird mit Medium MDI 1.m. gefüllt und mit der Vorkultur (Nr. 1) beimpft. Die Temperatur wird bei 30° C gehalten. Die Umdrehungsgeschwindigkeit des Rührers beträgt 200 upm, die Belüftung 0,1 $Nm^3/h$. Ohne weitere Veränderungen wird die Fermentation über 66 Stunden durchgeführt. Nach dieser Zeit ist eine OD (623 nm) von 1,1 und ein pH von 7,9 erreicht.

3. Hauptfermentation: Ein Bioreaktor mit 700 l Inhalt, Firma Giovanola, Monthey, Schweiz, mit Blattrührsystem wird mit Medium MDI 1.m. gefüllt. Nach dem Beimpfen mit der Vorfermentationen (Nr. 2) wird eine Umdrehungsgeschwindigkeit von 150 upm und eine Belüftung von 1 $Nm^3/h$ eingestellt. Durch Erhöhung der Belüftung und der Drehzahl wird der Sauerstoffpartialdruck im Verlaufe der Kultivierung oberhalb von 60 % Sättigung gehalten. Nach 50 Stunden ist eine OD von 1,3 und ein pH von 8 erreicht. Der Kulturüberstand enhält jetzt das Antibiotikum. Im Agardiffusionstest gegen Staph. aureus erhält man einen gerade noch sichtbaren Hemmhof (ca. 7 mm Durchmesser), wenn man den Kulturüberstand 1:64 verdünnt.

Beispiel 3: Isolierung und Reinigung der Saframycine

a) Aufarbeitung einer 700 Liter Fermentation: Zum Ende der Fermentation von Beispiel 2 b) werden 4,0 Liter XAD-Harz ER-180® (Fa. Röhm & Haas) in die Kulturbrühe eingebracht. Das Gemisch wird 5 Stunden langsam gerührt. Das Harz wird über ein Sieb von den Zellen, in denen sich kein Antibiotikum befindet, und vom Medium abgetrennt. Es wird in eine Chromatographiesäule überführt und in einem Zuge mit 12 Liter (1 Liter/Std.) Isopropanol eluiert. Der Antibiotika-Komplex wird mit den ersten 6 Liter Isopropanol zu über 90 % eluiert. Die Saframycine enthaltenden Fraktionen werden sofort mit 0,1% (v/v) Essigsäure versetzt und am Rotationsverdampfer in braunen Kolben eingeengt. Der eingeengte XAD-Extrakt wiegt 27 g.

Die Saframycine Mx 1 und Mx 2 sind basische, licht- und oxidationsempfindliche Hydrochinon/Chinon-Derivate, die nur bei pH-Werten kleiner als 7 stabil sind. Die Reindarstellung erfolgt somit weitgehend unter Lichtausschluss und bei pH-Werten um 5. Die Substanz-haltigen Fraktionen werden in braun eingefärbten Glasbehältern zwischengelagert, die Langzeitlagerung geschieht bei -70°C.

b) Reinigung der Saframycine: Der Rohextrakt wird in 300 ml 0,07 M Phosphatpuffer pH 5 gelöst und auf eine offene Säule aufgetragen, die mit 1 Liter gequelltem CM-Sephadex® C-25 der Fa. Pharmacia in 0.07 M Phosphatpuffer pH 5 gefüllt ist. Zunächst wird mit 2 Liter Phosphatpuffer alles nicht absorbierte Material eluiert. In einem weiteren Schritt wird mit 1 Liter 0,07 M Phosphatpuffer pH 5/0,2 M Natriumchlorid leicht adsorbiertes Material heruntergewaschen. Der Antibiotika-Komplex wird vollständig mit 2 Liter 0,07 M Phosphatpuffer pH 5/0,5 M NaCl von der Säule eluiert, was durch eine wandernde gelbliche Bande sichtbar wird. Nach Einengen des Eluats und mehrfacher Extraktion des Rückstandes mittels Ethanol werden ca. 2,5 g hochaktiven Materials erhalten. Dieses enthält noch Reste an Phosphat-Salz. Zur Entsalzung wird über eine Sephadex® LH-20 Säule mit Methanol und 1 % Ameisensäure oder Essigsäure als Laufmittel chromatographiert, wobei Phosphat durch Formiat beziehungsweise Acetat ausgetauscht wird. Im Eluat noch enthaltene basische Verunreinigungen wie 2-Phenylethylamin und 2-(4-Hydroxyphenyl)ethylamin werden durch eine Chromatographie an Sephadex® LH-20 mit Dichlormethan/Methanol 1:1/1% Ameisensäure oder Essigsäure eliminiert. Dabei werden 700 mg eines Produktes erhalten, das gemäss analytischer HPLC (Nucleosil® C-18, 7,5 µm, Methanol/Wasser 60:40/0,005 M Heptansulfonsäure) als Hauptprodukte die beiden Saframycine Mx 1 und Mx 2, ferner die Bis-chinon-Derivate Saframycin Mx 1 BC und Mx 2 BC und das Bis-hydrochinon-Derivat Mx 1 BHC enthält.

c) Trennung der Saframycine: Das Gemisch der Saframycine wird durch semi-präparative HPLC an HD-Sil® 18-10-60 (Fa. Organogen, 25 cm x 16 mm) mit Methanol/Wasser 1:1/0,5 % Triethylammoniumformiatpuffer pH 6,0 aufgetrennt. Bei einem Fluss von 25 ml/min eluiert Mx 1 nach 6,4 Min. und Mx 2 nach 9,2 Min. Beide Substanzen werden als zähe, rot-braune Oele erhalten. Die Nebenkomponente Mx 1 BHC eluiert vor Mx 1, die Nebenkomponenten Mx 1 BC und Mx 2 BC in dieser Reihenfolge nach Mx 2.

Beispiel 4: Charakterisierung der Saframycine

a) Saframycin Mx 1 und Mx 2

| | Saframycin Mx 1 ($R^1$ = OH) | Saframycin Mx 2 ($R^1$ = H) |
|---|---|---|
| Dünnschicht-Laufverhalten ($R_f$-Werte) | | |
| Kieselgel 60 F 254 Essigester | 0 | 0 |
| $CH_2Cl_2/CH_3OH$ 9:1 | 0,23 | 0,20 |
| $CH_3OH$ | 0,47 | 0,36 |
| RP-18 Nano-Sil $C_{18}$-100 $CH_3OH/H_2O$ 4:1 | 0,67 | 0,65 |

Farbreaktion auf DC identisch für beide Saframycine. Ninhydrin: rot-violett, Dragendorff: rot-braun, Hydroxylamin/Eisen(III)chlorid: intensiv gelb, 254 nm: Fluoreszenzlöschung.

| | | |
|---|---|---|
| HPLC-Retentionszeit auf HD-Sil 18-10-60 | | |
| $CH_3OH/H_2O$ 3:2/0,005 M $C_7H_{15}SO_3H$ | 4,97 min | 6,38 min |
| UV-Spektrum $\lambda_{max}$ (log ε), c = 2 in $CH_3OH$ | 273 nm (3,95) | 273 nm (3,97) |
| optische Drehung $[\alpha]_D^{25}$ | -70,7° | -119,8° |
| IR-Spektrum als Film, ν ($cm^{-1}$) (sh = Schulter) | 3200, 2900, 1657, 1619(sh), 1459, 1300, 1236, 1158. | 3200, 2900, 1680(sh), 1656, 1623(sh), 1461, 1236, 1159, 1122. |
| FAB-MS Negativionen-Bereich | m/e 568 (M-OH)-H | - |
| Positivionen-Bereich | - | m/e 569 $M-H^+$ |
| Elementaranalyse | Monophosphat•$H_2O$ | Monoformiat•$H_2O$ |
| Summenformel: | $C_{29}H_{43}N_4O_{14}P$ (702,7) | $C_{30}H_{42}N_4O_{11}$ (640,67) |
| Berechnet: | C 49,56 H 6,17 N 7,97 % | C 56,20 H 6,60 N 8,70 % |
| Gefunden: | C 50,17 H 6,33 N 7,66 % | C 56,36 H 6,70 N 8,94 % |

NMR-Spektrum: 15 mg Monophosphat in 0,5 ml $CD_3OD$

s = Singulett, d = Dublett, t = Triplett, q = Quartett, m = Multiplett, dd = Doppeldublett, ddd = Dreifachdublett

| $^1$H-NMR | Saframycin Mx 1 | | Saframycin Mx 2 | |
|---|---|---|---|---|
| Proton | (ppm) | Kopplungskonstanten (Hz) | (ppm) | Kopplungskonstanten (Hz) |
| 1-H | 4,33 s | (1-4b) 3,0; (1-22a) 0,5; (1-22b) 2,5; | 3,59 m | (1-4a) 1,5; (1-4b) 3,5; (1-22a) 3,6; (1-22b) 1,5; |
| 3-H | 3,50 ddd | (3-4a) 2,5; (3-4b) 11; (3-11) 1,0; | 3,12 ddd | (3-4a) 3; (3-4b) 11; (3-11) 3; |
| 4-$H_a$ | 3,12 dd | (4a-4b) 17,5; | 3,04 ddd | (4a-4b) 18; |
| 4-$H_b$ | 1,51 ddd | | 1,59 ddd | |
| 11-H | 4,75 dd | (11-13) 1,0; | 4,90 dd | (11-13) 1,5; |
| 12-$CH_3$ | 2,84 s | | 2,88 s | |
| 13-H | 4,20 s | (13-14) 2,8; (13-21) unter 0,5; | 4,13 d | |
| 14-H | 4,82 d | | 4,95 s | |
| 21-$H_a$ | 4,78 s | | 3,11 dd | (21a-21b) 13; (21a-13) 3; |
| 21-$H_b$ | - | | 3,71 d | |
| 22-$H_a$ | 3,77 dd | (22a-22b) 14,0; | 4,09 dd | (22a-22b) 14,5; |
| 22-$H_b$ | 3,25 dd | | 3,34 dd | |
| 24-H | 3,69 q | (24-25) 7,0; | 3,68 q | (24-25) 7,1; |
| 25-$H_3$ | 0,93 d | | 0,82 d | |
| 7-$OCH_3$ | 4,02 s | | 4,02 s | |
| 17-$OCH_3$ | 3,75 s | | 3,75 s | |
| 14-$OCH_3$ | 3,72 s | | 3,69 s | |
| 16-$CH_3$ | 2,25 s | | 2,26 s | |
| 6-$CH_3$ | 1,93 s | | 1,93 s | |

| $^{13}C$-NMR C-Atom | Saframycin Mx 1 (ppm) | Saframycin Mx 2 (ppm) |
|---|---|---|
| 1 | 54,63 d | 61,11 d |
| 3 | 51,80 d | 57,07 d |
| 4 | 25,60 t | 26,19 t |
| 5 | 186,95 s | 187,17 s |
| 6 | 129,33 s | 128,87 s |
| 7 | 157,23 s | 157,37 s |
| 8 | 182,77 s | 182,91 s |
| 9 | 143,10 s | 143,04 s |
| 10 | 138,43 s | 137,63 s |
| 11 | 58,19 d | 58,15 d |
| 12-$CH_3$ | 42,15 q | 41,39 q |
| 13 | 59,98 d | 59,38 d |
| 14 | 72,98 d | 75,39 d |
| 15 | 147,16 s | 146,58 s |
| 16 | 123,44 s | 123,36 s |
| 17 | 148,07 s | 147,75 s |
| 18 | 142,74 s | 143,04 s |
| 19 | 112,20 s | 113,04 s |
| 20 | 119,36 s | 120,75 s |
| 21 | 89,23 d | 54,44 t |
| 22 | 41,39 t | 39,36 t |
| 23 | 170,85 s | 170,65 s |
| 24 | 50,02 d | 49,85 d |
| 25 | 17,16 q | 17,08 q |
| 6-$CH_3$ | 8,76 q | 8,81 q |
| 16-$CH_3$ | 9,87 q | 9,98 q |
| 7-$OCH_3$ | 61,45 q | 61,04 q |
| 17-$OCH_3$ | 61,02 q | 61,44 q |
| 14-$OCH_3$ | 58,19 q | 58,07 q |

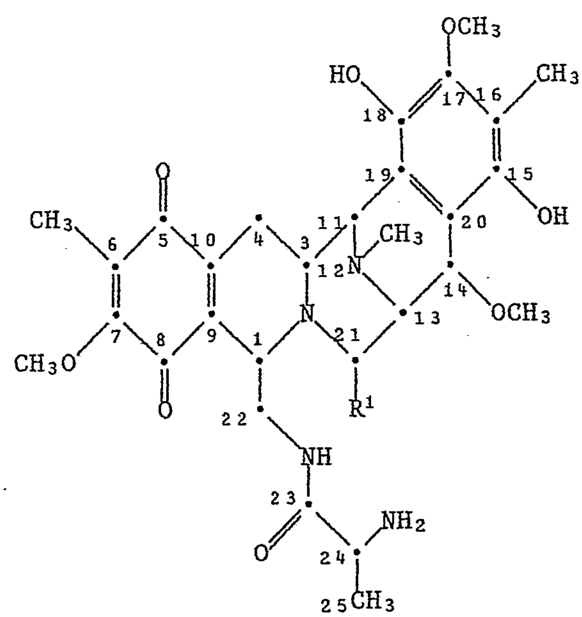

b) Saframycine Mx 1 BC, Mx 2 BC und Mx 1 BHC

| | Saframycin Mx 1 BC | Saframycin Mx 2 BC | Saframycin Mx 1 BHC |
|---|---|---|---|
| Dünnschicht-Laufverhalten ($R_f$-Werte) | | | |
| Kieselgel 60 F 254 Essigester | 0 | 0 | |
| $CH_2Cl_2/CH_3OH$ 9:1 | 0,52 | 0,53 | |
| $CH_3OH$ | 0,54 | 0,51 | |
| RP-18 Nano-Sil $C_{18}$-100 $CH_3OH/H_2O$ 4:1 | 0,61 | 0,56 | |
| HPLC-Retentionszeit auf HD-Sil 18-10-60 $CH_3OH/H_2O$ 3:2/0,005 M $C_7H_{15}SO_3H$ | 7,55 min | 10,86 min | 3,5 min |
| UV-Spektrum $\lambda_{max}$ (log ε) | 264 nm (4,32) | 264 nm (4,32) | 291 nm |
| $^1$H-NMR-Spektrum δ (ppm) in $CD_3OD$ | 4,05 und 4,04 (je s,3H, 7-$OCH_3$) und 17-$OCH_3$)<br>3,59 (s,3H, 14-$OCH_3$)<br>2,47 (s,3H, 12-$CH_3$)<br>2,00 (s,3H, 16-$CH_3$)<br>1,94 (s,3H, 6-$CH_3$)<br>1,28 (d,3H, 25-$H_3$) | 4,05 und 4,02 (je s,3H, 7-$OCH_3$ und 17-$OCH_3$)<br>3,59 (s,3H, 14-$OCH_3$)<br>2,46 (s,3H, 12-$CH_3$)<br>2,00 (s,3H, 16-$CH_3$)<br>1,94 (s,3H, 6-$CH_3$)<br>1,29 (d,3H, 25-$H_3$) | 3,78 (s,3H, 17-$OCH_3$)<br>3,70 (s,6H, 7-$OCH_3$ und 14-$OCH_3$)<br>2,95 (s,3H, 12-$CH_3$)<br>2,27 (s,3H, 16-$CH_3$)<br>2,17 (s,3H, 6-$CH_3$)<br>1,22 (d,3H, 25-$H_3$) |

Beispiel 5: Umwandlung der Saframycine

a) Oxidation von Saframycin Mx 1 zu Mx 1 BC mit Luftsauerstoff:

10 mg Saframycin Mx 1 (als Monophosphat) werden in 2 ml Phosphatpuffer pH 7,3 gelöst und 20 Min. im offenen Kolben gerührt. Mit verdünnter Phosphorsäure wird der pH-Wert auf ca. 6 eingestellt und die Lösung bei Oelpumpenvakuum eingeengt. Der Rückstand wird mehrmals mit Isopropanol aufgenommen, wobei die Puffersalze zurückbleiben. Die Isopropanollösung enthält 9,5 mg Saframycin Mx 1 BC.

Auf analoge Weise wird Saframycin Mx 2 zu Mx 2 BC aufoxidiert.

b) Reduktion von Saframycin Mx 1 zu Mx 1 BHC mit Wasserstoff:

In einem Hydriergefäss werden 10 mg Saframycin Mx 1 (als Monophosphat) in 0,5 ml Methanol und 0,5 ml Wasser gelöst, mit einer Spatelspitze Palladium-Aktivkohle (10 % Pd) versetzt und 10 Min. in einem schwachen Wasserstoffstrom geschüttelt. Danach wird der Katalysator unter einer Stickstoffatmosphäre abfiltriert und die Lösung, die 10 mg Mx 1 BHC enthält, eingeengt.

Das Saframycin Mx 1 BHC ist instabil und wird bei Gegenwart von Luftsauerstoff regiospezifisch zu Saframycin Mx 1 (Chinon/Hydrochinon) rückoxidiert.

c) Acetylierung von Saframycin Mx 1:

15 mg Saframycin Mx 1 werden in 2 ml Methanol und 2 ml Phosphatpuffer pH 7,3 gelöst, mit 0,05 ml Acetanhydrid versetzt und bei Raumtemperatur über Nacht gerührt. Die Reaktionslösung wird eingeengt, in wenig Wasser aufgenommen und auf eine XAD-2 Säule aufgetragen. Salze werden mit 2 Bettvolumina Wasser ausgewaschen. Danach wird das Produkt mit 1 Bettvolumen Methanol eluiert. Die Methanollösung enthaltend 15 mg Acetyl-Saframycin Mx 1 BC wird im Vakuum eingeengt.

UV-Spektrum in Methanol: $\lambda_{max}$ 266 nm, log $\varepsilon$ 4,32

FAB-MS im Positivionenbereich:
erwartet: m/e 627 (M + H)+
gefunden: m/e 613 (100 %), [M + 2xH - 1xO] + H+
m/e 615 (30 %), [M + 4xH - 1xO] + H+
Hochauflösung für $C_{31}H_{41}N_4O_9$ ber. 613,2873, gef. 613,2859.

$^1$H-NMR in $CD_3OD$: 4,05 und 4.02 (je s,3H, 7-$OCH_3$ und 17-$OCH_3$), 3,55 (s,3H, 14-$OCH_3$), 2,43 (s,3H, 12-$CH_3$), 2,00 (s,3H, 16-$CH_3$), 1,95 (s,3H, 6-$CH_3$), 1,75 (s,3H, Acetyl-$CH_3$), 1,10 (d,3H, 25-$H_3$).

Beispiel 6: Bestimmung der minimalen Hemmkonzentration der Saframycine Mx 1 und Mx 2

Die Testorganismen werden in einer Zelldichte von jeweils $10^5$ Zellen/ml (Myxococcus xanthus: $10^7$ Zellen/ml) in den entsprechenden Nährmedien in Reagenzgläsern vorgelegt.

Als Medien werden verwendet: Für Myxococcus xanthus: MD I 1.m. Für übrige Bakterien: Pepton aus Casein, tryptisch verdaut (Merck) 0,5 %; Proteose-Pepton (Difco) 0,5 %; Fleischextrakt (Oxoid) 0,1 % pH 7,0. Für Hefen: Bacto-Pepton (Difco) 1 %; Yeast Extract (Difco) 1 %, Glycerin 2 %, pH 6,3.

Konzentrationsreihen der in Methanol gelösten Safrmycine Mx 1 und Mx 2 werden zu den Zellsuspensionen der Testorganismen zugegeben und die Reagenzgläser 18 bis 40 Stunden bie 30°C bebrütet.

Die minimale Hemmkonzentration der Saframycine Mx 1 und Mx 2 sind in folgender Tabelle zusammengestellt:

| Testorganismus | Saframycin Mx 1 (µg/ml) | Saframycin Mx 2 (µg/ml) |
|---|---|---|
| Staphylococcus aureus GBF 16 | 0,004 | 0,5 |
| Bacillus subtilis DSM 10 | 0,063 | 1 |
| Micrococcus luteus DSM 348 | 0,001 | 0,063 |
| Escherichia coli CG 164 | 0,08 | 5 |
| Escherichia coli tol C | 0,32 | 20 |
| Proteus morganii CG 166 | 1,25 | >20 |
| Myxococcus xanthus Mx x48 | 20 | nicht getestet |
| Saccharomyces cerevisiae GBF 36 | >20 | >20 |

Gegenüber Staph. aureus weisen die Bis-chinone Mx 1 BC und Mx 2 BC ähnliche Aktivitäten auf wie Mx 1 und Mx 2. Das Bis-hydrochinon Mx 1 BHC ist unter den Testbedingungen nicht stabil.

Beispiel 7: Pharmazeutisches Präparat zur parenteralen Verabreichung

Je 5 ml einer sterilen wässrigen Lösung von 1 % Saframycin Mx 1 (als Phosphat-Monohydrat) werden unter aseptischen Bedingungen in 5 ml-Ampullen oder Vials eingefüllt und gefriergetrocknet. Die Ampullen bzw. Vials werden unter Stickstoff verschlossen und geprüft.

Lösungen von Saframycin Mx 2, Mx 1 BC, Mx 2 BC, Mx 1 BHC und Acetyl-Mx 1 BC werden auf gleiche Art und Weise verarbeitet.

**Patentansprüche**

1. Verbindungen der Formel

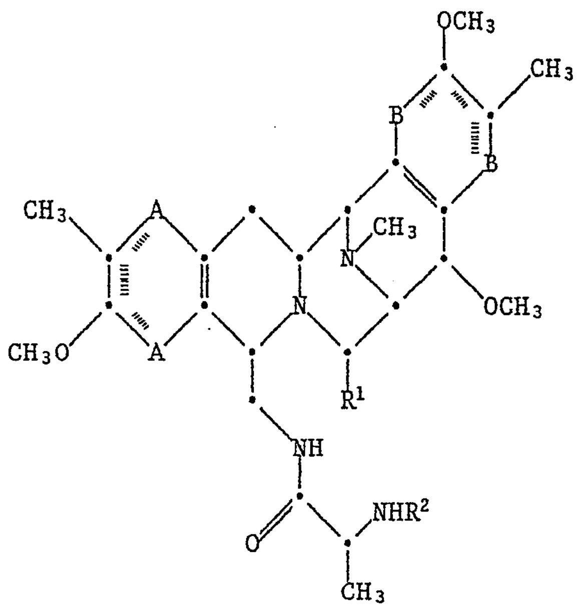

(I),

worin $R^1$ Wasserstoff oder Hydroxy, $R^2$ Wasserstoff oder Acyl und A und B unabhängig voneinander C=O oder C-OH bedeuten, wobei die gestrichelte Linie eine C=C-Doppelbindung an der Stelle der mittleren Bindung, wenn A oder B C=O sind, oder an der Stelle der beiden äusseren Bindungen, wenn A oder B C-OH sind, symbolisiert, und Salze solcher Verbindungen.

2. Verbindungen gemäss Anspruch 1, die die räumliche Struktur gemäss Formel

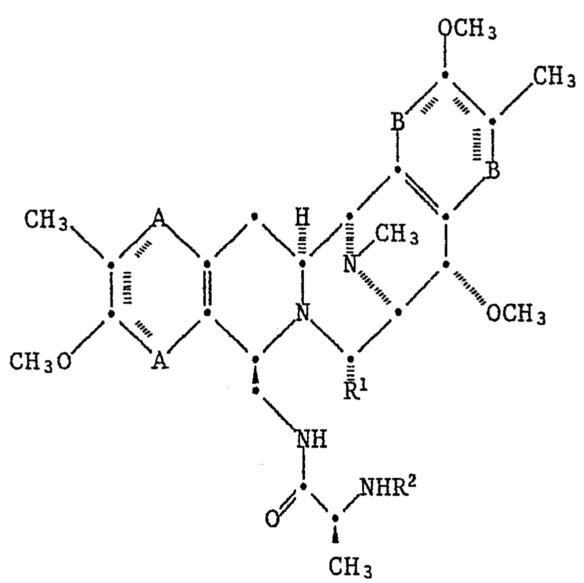

(II)

aufweisen.

3. Verbindungen der Formel I gemäss Anspruch 1 oder 2, worin $R^1$ Wasserstoff oder Hydroxy, $R^2$ Wasserstoff, A C=O und B C=O oder C-OH bedeuten und die gestrichelte Linie die obengenannte Bedeutung hat, und pharmazeutisch verwendbare Salze solcher Verbindungen.

4. Verbindung der Formel I gemäss Anspruch 1 oder 2, worin $R^1$ Hydroxy, $R^2$ Wasserstoff, A C-OH und B C-OH bedeuten und die gestrichelte Linie die obengenannte Bedeutung hat, und pharmazeutisch verwendbare Salze dieser Verbindung.

5. Verbindung der Formel I gemäss Anspruch 1 oder 2, worin $R^1$ Hydroxy, $R^2$ Acetyl, A C=O und B C=O bedeuten und die gestrichelte Linie die obengenannte Bedeutung hat, und pharmazeutisch verwendbare Salze dieser Verbindung.

6. Verbindungen der Formel I gemäss Anspruch 1 oder 2, worin $R^1$ Wasserstoff oder Hydroxy, $R^2$ Wasserstoff, A C=O und B C-OH bedeuten und die gestrichelte Linie die obengenannte Bedeutung hat, und pharmazeutisch verwendbare Salze solcher Verbindungen.

7. Verfahren zur Herstellung von Verbindungen der Formel I gemäss Anspruch 1, dadurch gekennzeichnet, dass man den Stamm Mx x48 der Species Myxococcus xanthus oder eine von diesem abgeleitete, die Verbindungen der Formel I produzierende Mutante in einer Kultur enthaltend Kohlenstoff- und Stickstoffverbindungen und essentielle anorganische Salze in leicht assimilierbarer Form bei einer Temperatur zwischen 15°C und 40°C und einem pH-Wert zwischen 5 und 9 unter aeroben Bedingungen züchtet, die gebildeten Verbindungen der Formel I isoliert und, wenn erwünscht, eine erhältliche Verbindung der Formel I in eine andere Verbindung der Formel I und/oder eine erhältliche Verbindung in ein Salz und/oder ein erhältliches Salz in die freie Verbindung oder in ein anderes Salz überführt.

8. Verfahren gemäss Anspruch 7, dadurch gekennzeichnet, dass zur Isolierung der Verbindungen der Formel I aus der rohen Fermentationsbrühe diese an makroporöse, nicht-ionische Harze adsorbiert und durch chromatographische Methoden gereinigt werden.

9. Verfahren gemäss Anspruch 7 oder 8, dadurch gekennzeichnet, dass die Verbindungen der Formel I durch Chromatographie an einem Ionenaustauscher mit schwach sauren funktionellen Gruppen, Adsorptions- bzw. Verteilungschromatographie und/oder Hochdruck-Flüssigchromatographie an unpolaren Oberflächen gereinigt werden.

10. Die nach dem Verfahren gemäss Anspruch 7 erhältlichen antibiotisch wirksamen Verbindungen.

11. Der Stamm Mx x48 des Mikroorganismus Myxococcus xanthus, der bei der National Collection of Industrial and Marine Bacteria, Aberdeen, Schottland, unter der Nummer NCIB 12 268 hinterlegt ist, oder eine von diesem Stamm abgeleitete Mutante, die eine Verbindung der Formel I produziert.

12. Pharmazeutische Präparate enthaltend Verbindungen der Formel I oder pharmazeutisch verwendbare Salze davon gemäss Anspruch 1 im Gemisch mit pharmazeutisch verwendbaren Trägerstoffen.

13. Verwendung von Verbindungen der Formel I oder von pharmazeutisch verwendbaren Salzen davon gemäss Anspruch 1 zur Herstellung von pharmazeutischen Präparaten.

Patentansprüche für die folgenden Vertragstaaten: AT, ES, GR

1. Verfahren zur Herstellung von Verbindungen der Formel

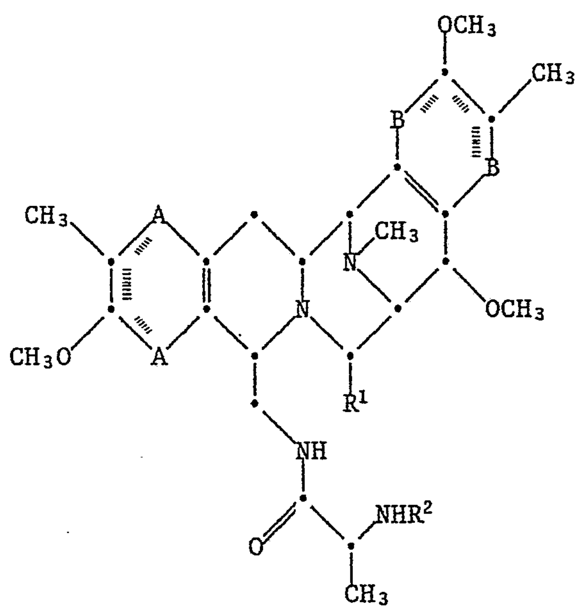

(I),

worin $R^1$ Wasserstoff oder Hydroxy, $R^2$ Wasserstoff oder Acyl und A und B unabhängig voneinander

C=O oder C-OH bedeuten, wobei die gestrichelte Linie eine C=C-Doppelbindung an der Stelle der mittleren Bindung, wenn A oder B C=O sind, oder an der Stelle der beiden äusseren Bindungen, wenn A oder B C-OH sind, symbolisiert, und von Salzen solcher Verbindungen, dadurch gekennzeichnet, dass man den Stamm Mx x48 der Species Myxococcus xanthus oder eine von diesem abgeleitete, die Verbindungen der Formel I produzierende Mutante in einer Kultur enthaltend Kohlenstoff- und Stickstoffverbindungen und essentielle anorganischen Salze in leicht assimilierbarer Form bei einer Temperatur zwischen 15° C und 40° C und einem pH-Wert zwischen 5 und 9 unter aeroben Bedingungen züchtet, die gebildeten Verbindungen der Formel I isoliert und, wenn erwünscht, eine erhältliche Verbindung der Formel I in eine andere Verbindung der Formel I und/oder eine erhältliche Verbindung in ein Salz und/oder ein erhältliches Salz in die freie Verbindung oder in ein anderes Salz überführt.

2. Verfahren gemäss Anspruch 1, dadurch gekennzeichnet, dass zur Isolierung der Verbindungen der Formel I aus der rohen Fermentationsbrühe diese an makroporöse, nicht-ionische Harze adsorbiert und durch chromatographische Methoden gereinigt werden.

3. Verfahren gemäss Anspruch 1 oder 2, dadurch gekennzeichnet, dass die Verbindungen der Formel I durch Chromatographie an einem Ionenaustauscher mit schwach sauren funktionellen Gruppen, Adsorptions- bzw. Verteilungschromatographie und/oder Hochdruck-Flüssigchromatographie an unpolaren Oberflächen gereinigt werden.

4. Verfahren gemäss Anspruch 1, 2 oder 3 zur Herstellung von Verbindungen, die die räumliche Struktur gemäss Formel

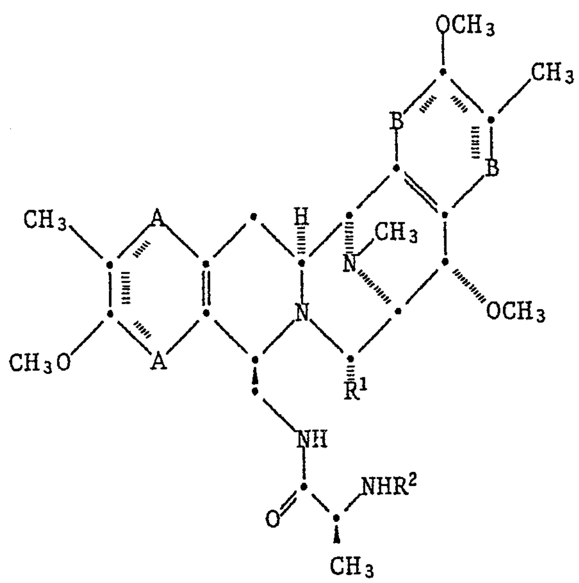

(II)

aufweisen.

5. Verfahren gemäss Anspruch 1, 2 oder 3 zur Herstellung von Verbindungen der Formel I, worin $R^1$ Wasserstoff oder Hydroxy, $R^2$ Wasserstoff, A C=O und B C=O oder C-OH bedeuten und die gestrichelte Linie die obengenannte Bedeutung hat, und von pharmazeutisch verwendbaren Salzen solcher Verbindungen.

6. Verfahren gemäss Anspruch 1, 2 oder 3 zur Herstellung der Verbindung der Formel I, worin $R^1$ Hydroxy, $R^2$ Wasserstoff, A C-OH und B C-OH bedeuten und die gestrichelte Linie die obengenannte Bedeutung hat, und von pharmazeutisch verwendbaren Salzen dieser Verbindung.

7. Verfahren gemäss Anspruch 1, 2 oder 3 zur Herstellung der Verbindung der Formel I, worin $R^1$ Hydroxy, $R^2$ Acetyl, A C=O und B C=O bedeuten und die gestrichelte Linie die obengenannte Bedeutung hat, und von pharmazeutisch verwendbaren Salzen dieser Verbindung.

8. Verfahren gemäss Anspruch 1, 2 oder 3 zur Herstellung der Verbindungen der Formel I, worin $R^1$ Wasserstoff oder Hydroxy, $R^2$ Wasserstoff, A C=O und B C-OH bedeuten und die gestrichelte Linie die obengenannte Bedeutung hat, und von pharmazeutisch verwendbaren Salzen solcher Verbindungen.

9. Der Stamm Mx x48 des Mikroorganismus Myxococcus xanthus, der bei der National Collection of Industrial and Marine Bacteria, Aberdeen, Schottland, unter der Nummer NCIB 12 268 hinterlegt ist, oder eine von diesem Stamm abgeleitete Mutante, die eine Verbindung der Formel I produziert.

10. Verfahren zur Herstellung von pharmazeutischen Präparaten enthaltend Verbindungen der Formel I oder pharmazeutisch verwendbare Salze davon gemäss Anspruch 1 im Gemisch mit pharmazeutisch verwendbaren Trägerstoffen.

Europäisches Patentamt

# EUROPÄISCHER RECHERCHENBERICHT

Nummer der Anmeldung

EP 87 81 0454

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| Y | WO-A-8 501 049 (YOSHITOMI)<br>* Zusammenfassung *<br>--- | 1,12 | C 07 D 471/18<br>C 12 P 17/18<br>A 61 K 31/495//<br>(C 07 D 471/18<br>C 07 D 241:00<br>C 07 D 221:00<br>C 07 D 221:00 )<br>(C 12 P 17/18<br>C 12 R 1:01 ) |
| Y | TETRAHEDRON LETTERS, Nr. 25, 1979, Seiten 2355-2358, Pergamon Press Ltd, Oxford, GB; T. ARAI: "The structures of novel antibiotics, saframycin B and C"<br>* Formel 3; Seite 2356, Zeilen 3-5 *<br>----- | 1,12 | |
| | | | RECHERCHIERTE SACHGEBIETE (Int. Cl.4) |
| | | | C 07 D 471/00<br>A 61 K 31/00<br>C 12 P 17/00 |

Der vorliegende Recherchenbericht wurde für alle Patentansprüche erstellt

| Recherchenort | Abschlußdatum der Recherche | Prüfer |
|---|---|---|
| DEN HAAG | 26-11-1987 | ALFARO I. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
& : Mitglied der gleichen Patentfamilie, übereinstimmendes Dokument

EPO FORM 1503 03.82 (P0403)